# EUROPEAN PATENT APPLICATION

(11) **EP 4 837 575 A1**
(43) Date of publication of application: **11.11.2026**
(21) Application number: 25174364.7
(22) Date of filing: 06.05.2025
(51) Int. Cl.: A61K 31/496, A61K 31/7052, A61P 3/10

(54) **COMPOUNDS FOR TREATING IMPAIRED INSULIN SECRETION AND TYPE 2 DIABETES**

(71) Applicant: AcroPharma AB, 222 29 Lund (SE)
(72) Inventor: SALEHI, Albert, 226 54 Lund (SE); KHAYYAMI, Masoud, 22239 Lund (SE)
(74) Representative: KIPA AB

(57) **Abstract**

The disclosure provides an agent selected from Itraconazole and Azithromycin, or a pharmaceutically acceptable salt or analogue thereof, for use in the treatment of impaired insulin secretion in a human subject. The disclosure also provides agent selected from Itraconazole and Azithromycin, or a pharmaceutically acceptable salt or analogue thereof, for use in the treatment or prevention of type 2 diabetes in a human subject. Methods of treatment of type 2 diabetes and progression of a prediabetic condition are also provided.

## Description

### FIELD

The present invention relates to agents and methods for treating impaired insulin secretion in human subjects. The invention further relates to agents and methods to treat type 2 diabetes in human subjects.

### INTRODUCTION

Type 2 diabetes, also known as adult-onset diabetes, is a form of diabetes mellitus in which patients have high blood sugar levels, insulin resistance and a relative lack of insulin due to impaired insulin secretion. It is a major public health problem due to the high number of affected patients, with more than 390 million people affected world-wide in 2015. Type 2 diabetes is now considered an epidemic, with a rapid increase in prevalence world-wide.

Type 2 diabetes accounts for about 90-95% of all diabetes cases. Many factors can potentially give rise to or exacerbate type 2 diabetes. These include hypertension, elevated cholesterol, metabolic syndrome and overweight/obesity. As an example, approximately 90% of patients with type 2 diabetes are overweight/obese. Current treatments consist mainly of glucose-lowering medications or insulin sensitizers.

Insulin is a hormone involved in glucose homeostasis, together with glucagon. In response to rising levels of blood glucose, insulin is produced by pancreatic beta cells located in the islets of Langerhans. Thus, glucose is taken up from the blood by hepatocytes, muscle cells, and adipocytes used either as an energy source or for storage as glycogen and triglycerides. It also inhibits lipolysis, preventing fatty acid release from fat tissues. On the contrary, low blood glucose levels result in both reduced production and release of insulin. Together with glucagon action, it results in glucose release into the bloodstream. In pathological situations, either insulin production by beta-cells is not sufficient (type 1 diabetes) and/or cells respond poorly to it (insulin resistance; type 2 diabetes), leading to persistent high levels of blood glucose. Precise mechanisms involved in these pathologies are not yet completely understood.

Type 2 diabetes mellitus is considered to be a complex metabolic disorder. It results from the combination of impaired pancreatic insulin secretion due to beta-cells dysfunction and insulin resistance as well as damaged glucagon secretion. Impairment of glucose-stimulated production of insulin involves a progressive loss of pancreatic beta-cells as well as a decline in islet cell function. Insulin resistance consists for example of suppressed or reduced effects of insulin in peripheral organs/tissues (liver, muscles and fat tissues) or enhanced lipolysis in adipocytes, leading to increased circulation of free fatty acids. These events result in increased endogenous glucose production by the liver together with decreased glucose uptake due to reduced insulin receptor expression, defects in post-receptor actions of insulin, hepatic glucose overproduction or blocking of insulin-signaling pathways. Insulin resistance is a hallmark of a more complex syndrome, called metabolic syndrome, which is a grouping of risk factors for coronary heart disease and diabetes mellitus including abdominal obesity, elevated triglyceride levels, decreased high-density lipoprotein levels, elevated blood pressure, and elevated fasting plasma glucose levels.

Persistent high blood glucose leads to both acute and chronic complications that may be very disabling, even fatal for diabetic patients, such as heart disease and stroke, which are the most life-threatening consequences of diabetes mellitus. Long-term persistent elevated blood glucose damages blood vessels, leading to microvascular and macrovascular angiopathy, which account for most of the increased morbidity and mortality associated with the disease. Microvascular complications are responsible for diabetic cardiomyopathy and nephropathy, both sometimes leading to organ failure, retinopathy, which can lead to severe vision loss, and neuropathy. Macrovascular complications rather concern cardiovascular impairments that are responsible for coronary artery disease that in the end provokes angina or myocardial infarction, diabetic myonecrosis, peripheral vascular disease and stroke. Macrovascular complications are more common and up to 80% of patients with type 2 diabetes will develop or die of a macrovascular disease.

Unfortunately, existing treatments do not succeed in restoring normoglycemia in the long term, since beta-cell function declines over time. Moreover, there is presently no single drug able to reverse all aspects of the disease.

Insulin may also be administered to type 2 diabetes patients when glucose-lowering drugs and diet fail to control glycemia. Use of insulin, however, comprises side effects including hypoglycemia when dosage is not appropriate, increased risk of developing colorectal cancer and weight gain, which is not recommended for diabetic patients, particularly obese ones.

The progressive nature of type 2 diabetes implies that many patients will eventually require a combination of antidiabetics, possibly together with insulin. Antidiabetics have been developed in order to counteract the main mechanisms involved in type 2 diabetes: insulin resistance (biguanides and thiazolidinediones) and insulin secretion (sulfonylureas, glinides, dipeptidylpeptidase-4 inhibitors, and glucagon-like peptide 1 receptor agonists), in addition to particular mechanisms dealing with delayed absorption of glucose by the gastrointestinal tract. However, most of these medications have been shown to have deleterious side effects such as weight gain, peripheral edema or congestive heart failure and to loss in efficiency in a long term use.

### SUMMARY

The present disclosure seeks to provide solutions to overcome the foregoing limitations or disadvantages of the prior art.

In an aspect, the present disclosure provides an agent selected from Itraconazole and Azithromycin, or a pharmaceutically acceptable salt or analogue thereof, for use in the treatment of impaired insulin secretion in a human subject.

In another aspect, the present disclosure provides an agent selected from Itraconazole and Azithromycin, or a pharmaceutically acceptable salt or analogue thereof, for use in the treatment or prevention of type 2 diabetes in a human subject

The present disclosure further provides a method of treatment of impaired insulin secretion and a method of treatment of type 2 diabetes in human subjects..

Thus, in an aspect the disclosure provides a method of treating impaired insulin secretion in a human subject in need thereof, the method comprising administering an agent selected from Itraconazole and Azithromycin, or a pharmaceutically acceptable salt or analogue thereof, to the human subject.

In another aspect the disclosure provides a method of treatment or prevention of type 2 diabetes in a human subject in need thereof, the method comprising administering an agent selected from Itraconazole and Azithromycin, or a pharmaceutically acceptable salt or analogue thereof, to the human subject.

Additional features and advantages are described, and will be apparent from, the following detailed description and the figures.

### BRIEF DESCRIPTION OF THE DRAWINGS

FIG. 1 shows the dose-response effect of Itraconazole on the insulin secretion from isolated mouse pancreatic islets.
FIG. 2 shows the dose-response effect of Azithromycin on the insulin secretion from isolated mouse pancreatic islets.
FIG. 3 shows how the effect of High K⁺ on glucose-stimulated insulin secretion is further increased by Itraconazole.

### DESCRIPTION

The present disclosure is based on the surprising finding that certain antibacterial and antifungal agents have activity to rescue impaired insulin secretion. Specifically, the present inventor has found that Itraconazole, a known member of the N-arylpiperazine family of compounds, and Azithromycin, a well-known macrolide, have a significant effect on impaired insulin secretion. As a consequence, ltranconazole and Azithromycin and analogues thereof, are believed to have activity to treat impaired insulin secretion in humans. Therefore, these compounds also have activity to treat or prevent development of type 2 diabetes in human subjects.

Although itraconazole is known to be used to treat fungal infection in patients with type 2 diabetes and azithromycin is known used to treat bacterial infection in type 2 diabetes patients, the activity of these compounds to remedy deficient insulin secretion is highly surprising. Furthermore, without intending to be limited by theory, the mechanism of action of these compounds to ameliorate impaired insulin secretion is believed to be unrelated to their mechanism of action as antibacterial and antifungal agents.

Thus, through the action of these compounds on the insulin secretion pathway, a novel path to treat impaired insulin secretion to either prevent development of type 2 diabetes in prediabetic patients, or treat type 2 diabetes in diagnosed patients, is provided by the present disclosure.

Thus, an individual determined to have impaired insulin secretion can have a diagnosis of type 2 diabetes. Alternatively, the individual does not have a diagnosis of impaired insulin secretion, in which case the impaired insulin secretion is indicative of the individual having a prediabetic condition.

The data shown in Examples 1 and 2 herein establish the dose-dependent activity of itraconazole and azithromycin to potentiate glucose-stimulated insulin secretion in isolated mouse pancreatic islets. The dramatic effect on insulin secretion shows the potential of these compounds to treat or prevent type 2 diabetes in humans.

Further, a comparison of the effect of Itraconazole compared with Glibenclamide, a well known second-generation sulfonylurea known to have a prolonged hypglycemic effect, on insulin secretion has been tested.

As the results show (Example 3), only Itraconazole can further increase the potentiation of glucose-stimulated insulin secretion by K⁺/diazoxide. This indicates that there is different mechanism of action of Itraconazole compared with Glibenclamide on the potentiation of insulin secretion, which underpins the novel mechanistic and biological novelty of the mode of action of itraconazole and azithromycin on insulin secretion.

In the present context, the term "analogue" refers to compounds or agents that have structural and functional similarity. Thus, an analogue may have a structure that, compared to the reference compound or agent, differs in one or more atoms, functional groups or substructures that are replaced by alternate atoms, functional groups or substructures. The analogue also has a similar or identical activity, in particular activity with respect to the ability to increase impaired insulin secretion in humans.

Itraconazole, sometimes abbreviated as ITZ, is an antifungal compound used to treat fungal infections. The compound, described in US 4,267,179, has also been explored as an anticancer agent.

Itraconazole is defined as (±)-1-sec-butyl-4-[p-[4-[p-[[(2R*,4S*)-2-(2,4-dichlorophenyl)-2-(1H-1,2,4-triazol-1-ylmethyl)-1,3-dioxolan-4-yl]methoxy]phenyl]-1-piperazinyl]phenyl]-Δ²-1,2,4-triazolin-5-one, or alternatively, as 4-[4-[4-[4-[[2-(2,4-dichlorophenyl)-2-(1H-1,2,4-triazol-1-ylmethyl)-1,3-dioxolan-4-yl]methoxy]phenyl]-1-piperazinyl]phenyl]-2,4-dihydro-2-(1-methylpropyl)-3H-1,2,4-triazol-3-one. The chemical structure of itraconazole is shown in the following formula I:

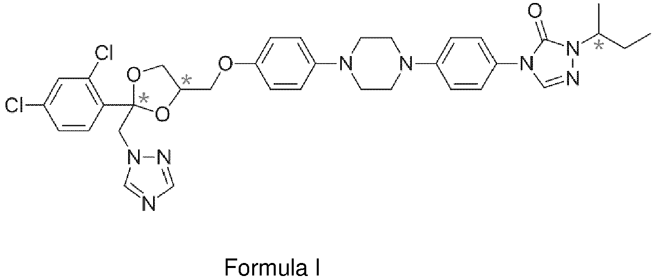

There are three asymmetric carbons in itraconazole, indicated by an asterisk in Formula I: one in the sec-butyl side chain on the triazolone and two in the dioxolane ring. As a result, eight possible stereoisomers of itraconazole exist: (R,R,R), (S,S,S), (R,R,S), (S,S,R), (R,S,S), (R,S,R), (S,R,S), and (S,R,R).

(±)Cis-ltraconazole comprises a mixture of only those isomers that describe a "cis" relationship in the dioxolane ring, i.e., the (1H-1,2,4-triazol-1-ylmethyl) moiety and the substituted phenoxy moiety are located on the same side of a plane defined by the 1, 3-dioxolane ring. By convention, the first represented chiral center is at C-2 of the dioxolane ring, the second is at C-4 of the dioxolane ring, and the third is in the sec-butyl group. Hence, (±)cis-itraconazole is a mixture of (R,S,S), (R,S,R), (S,R,S) and (S,R,R) isomers.

The four possible stereoisomeric cis forms of itraconazole, and diastereomeric pairs thereof, are described in more detail in U.S. 5,474,997 and U.S. 5,998,413. In general, the individual stereoisomeric forms of cis-itraconazole have antifungal properties, and contribute to the overall activity of (±)cis-itraconazole. Thus, in the present context, all the stereoisomeric forms of cis-itraconazole are contemplated to have the claimed activity to potentiate insulin secretion in humans. Accordingly, a clinical formulation of itraconazole can contain a 1:1:1:1 mixture of the four stereoisomers (two enantiomeric pairs).

Azithromycin, also known as 9-deoxy-9α-aza-9α-methyl-9α-homoerythromycin A, is a macrolide compound having antibiotic activity and is used to treat infections such as ear infections, strep throat, pneumonia, diarrhea and certain intestinal infections. The chemical structure of azithromycin is shown in the below Formula II:

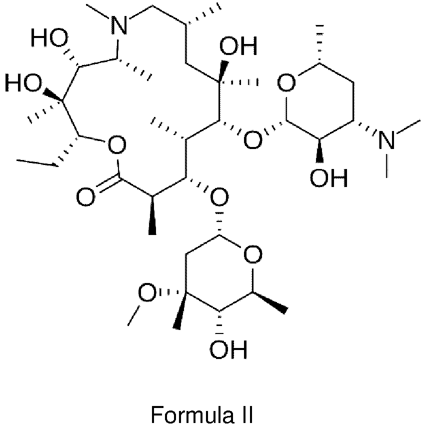

Azithromycin is sometimes also used to treat malaria. Furthermore, the compound has been used to treat airway diseases such as asthma and chronic obstructive pulmonary disease (COPD). The compound has also been explored for its potential role in treating cystic fibrosis and chronic fatigue syndrome.

The antibacterial mechanism of action of azithromycin is believed to originate in its interfering with protein synthesis, binding to the 50S subunit of the bacterial ribosome, inhibiting mRNA translation. Some research indicates that the compound has antiinflammatory and immunomodulatory properties, which are believed to be exhibited through its suppression of proinflammatory cytokines and enhancing the production of antiinflammatory cytokines, which is important in dampening inflammation.

Analagoues of the compounds Itraconazole and Azithromycin are compounds that, by virtue of their molecular and/physiochemical properties, is likely to have the same or similar biological activity. An analogue in this context may be largely isoelectronic to the reference compound (Itraconazole and/or Azithromycin).

For example, analogues of Itraconazole that have activity to potentiate insulin secretion may be selected from Voriconazole, Posaconazole, Isavuconazole, Fluconazole, Ravuconazole and Ketoconazole.

Analogues of Azithromycin that have activity to potentiate insulin secretion may be selected from clarithromycin, dirithromycin, erythromycin, carbomycin A, josamycin, kitasamycin, midecamycin, oleandomycin, spiramycin, troleandomycin, tylosin, roxithromycin and boromycin.

These agents are therefore contemplated to have activity to treat or ameliorate impaired insulin secretion or treat or prevent development of, type 2 diabetes, in particular in individuals with a prediabetic condition.

An individual with impaired insulin secretion may have a prediabetic condition that is further characterized by the presence of one or more of: elevated fasting blood glucose levels, elevated glucose levels in a glucose tolerance test or elevated HbA1c in blood.

Fasting glucose levels are normally below 100 mg/dL in blood. A measurement of 100 - 125 mg/dL is considered prediabetic, while measurements of greater than 125 mg/dL are considered diabetic (hyperglycemia).

A glucose tolerance test, also referred to as a oral glucose tolerance test, measures the body's response to glucose. The test measures the ability of the body to move sugar from the blood into tissues such as muscles and fat tissue, i.e. it measures the ability of the body to use and store glucose. In the test, a fixed amount of glucose is administered and the glucose levels in blood measured after a certain period of time.

In glucose tolerance tests, a healthy blood glucose level is considered less than 140mg/dL (equivalent to 7.8 mmol/L) 2h after administration of 75g sugar (glucose). A prediabetic condition is characterized by glucose levels that are above 140mg/dL and less than 200 mg/dL 2h after administration of 75g sugar. A value of 200 mg/dL or greater is considered suggestive of diabetes.

Glycated haemoglobin (HbA1c) is a predictive marker for diabetes or a prediabetic condition. This is because glucose sticks to haemoglobin in blood and the measure of the relative quantity of glycated haemoglobin is therefore a measure of the exposure to high blood glucose levels. A test for HbA1c determines the percentage of red blood cells that have glycoated haemoglobin.

Normal HbA1c levels in humans are 5-5.6%. A value in the range of 5.7 - 6.4% is considered consistent with a prediabetic condition. Elevated HbA1c in blood can be consistent with a diabetic if the levels are at 6.5% or higher (48 mmol/mol or higher).

A diagnosis of type 2 diabetes is thus typically made if one or more of the following results are found in a blood test:
- Increased HbA1c, a measurement of 6.5% or higher, preferably in two separate tests performed at two different times
- Fasting blood glucose levels of greater than 125 mg/dL
- Glucose tolerance test with a result of 200mg/dL or higher.

Accordingly, an individual having a prediabetic condition by one or more of these criteria may be suitable for administration of Itraconazole and Azithromycin, or a pharmaceutically acceptable salt or analogue thereof, to treat the prediabetic condition.

The agents described herein can be used to treat type 2 diabetes in a human subject. The agents can also, or alternatively, be used to prevent development of type 2 diabetes in a human subject. Prevention of development of type 2 diabetes is especially important and useful for individuals with prediabetic symptoms, or diagnosis of a prediabetic condition.

Accordingly, Itraconazole and Azithromycin, or a pharmaceutically acceptable salt or analogue thereof, can be used to prevent, or reduce the risk of, the progression of a prediabetic condition to type 2 diabetes in the human subject. The prediabetic condition may be manifested by the presence of one or more of: elevated fasting blood glucose levels, elevated glucose levels in a glucose tolerance test or elevated HbA1c in blood.

The agents disclosed herein can be administered using classical routes of administration, including oral, intravenous, intramuscular or subcutaneous administration. Alternate routes are also possible, including transdermal, pulmonary, nasal, and buccal routes.

The agents can be formulated for the intended use as known in the art. For example, the agents can be formulated for oral administration as a capsule, tablet, or suspended powder (liquid suspension), or as a liquid solution of the agent or a pharmaceutically acceptable salt, analogue or stereoisomer thereof. Dosing may be once a day, twice daily or other appropriate dosing.

As used herein, including in the claims, singular forms of terms are to be construed as also including the plural form and vice versa, unless the context indicates otherwise. Thus, it should be noted that as used herein, the singular forms "a," "an," and "the" include plural references unless the context clearly dictates otherwise.

Throughout the description and claims, the terms "comprise", "including", "having", and "contain" and their variations should be understood as meaning "including but not limited to" and are not intended to exclude other components.

The present invention also covers the exact terms, features, values and ranges etc. in case these terms, features, values and ranges etc. are used in conjunction with terms such as about, around, generally, substantially, essentially, at least etc. (i.e., "about 3" shall also cover exactly 3 or "substantially constant" shall also cover exactly constant).

It will be appreciated that variations to the foregoing embodiments of the invention can be made while still falling within the scope of the invention n. Features disclosed in the specification, unless stated otherwise, can be replaced by alternative features serving the same, equivalent or similar purpose. Thus, unless stated otherwise, each feature disclosed represents one example of a generic series of equivalent or similar features.

Use of exemplary language, such as "for instance", "such as", "for example" and the like, is merely intended to better illustrate the invention and does not indicate a limitation on the scope of the invention unless so claimed. Any steps described in the specification may be performed in any order or simultaneously, unless the context clearly indicates otherwise.

All the features and/or steps disclosed in the specification can be combined in any combination, except for combinations where at least some of the features and/or steps are mutually exclusive. In particular, preferred features of the invention are applicable to all aspects of the invention and may be used in any combination.

### Example 1

The dose-response effect of Itraconazole on insulin secretion from isolated mouse pancreatic islets was studied. The islets were incubated at 1 mM (1G) or 16.7 mM glucose (16.7G) in the presence of increasing concentrations of Itraconazole for 60 min.

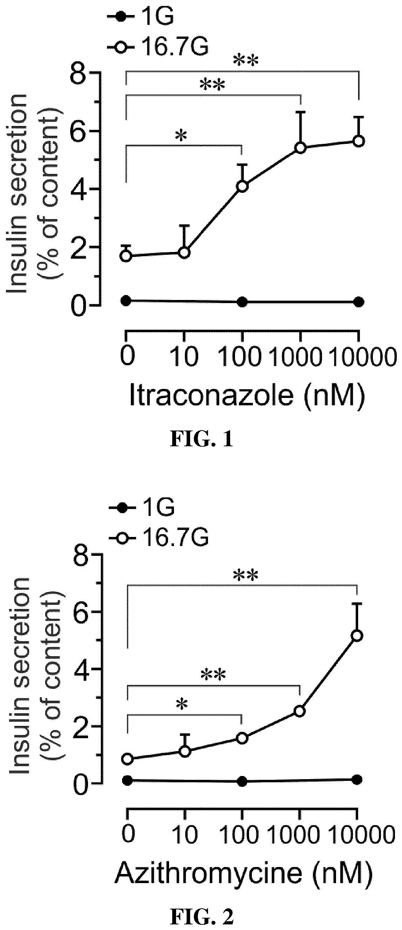

The results, displayed in FIG. 1 show that the presence of Itraconazole during incubation dose-dependently potentiated glucose-stimulated insulin secretion. Itraconazole did not affect insulin release at 1 mM glucose. Moreover, the effect is pronounced at low concentrations (50% of the rise in insulin secretion obtained at about 100nM concentration).

Shown is mean±SD for four repeated experiments in each group. *p<0.05, **p<0.01.

### Example 2

The dose-response effect of Azithromycin on the insulin secretion from isolated mouse pancreatic islets was studied. The islets were incubated at 1 mM (1G) or 16.7 mM glucose (16.7G) in the presence of increasing concentrations of Azithromycin for 60 min.

The results, displayed in FIG. 2 show Azithromycin dose-dependently potentiated glucose-stimulated insulin secretion. The effect becomes significant at low (about 1mM) concentrations. Azithromycin did not affect insulin release at 1 mM glucose.

Shown is mean±SD for four repeated experiments in each group. *p<0.05, **p<0.01.

### Example 3

The effect of Itraconazole on insulin secretion in glucose-stimulated cells in the presence of high K⁺ was tested.

Human β-cell line (EndoC βH1 cells) were incubated at 1 mM or 16.7 mM glucose in the presence of K⁺ (35 mM) and diazoxide (200 uM) to induce membrane depolarization by bypassing the membrane K⁺_{ATP} channels, in the presence or absence of Itraconazole (10 uM) or Glibenclamide (10 uM), a well known second-generation sulfonylurea antidiabetic agent having a prolonged hypoglycemic effect, for 60 min. Thereafter, the insulin secretory pattern of the agents was recorded.

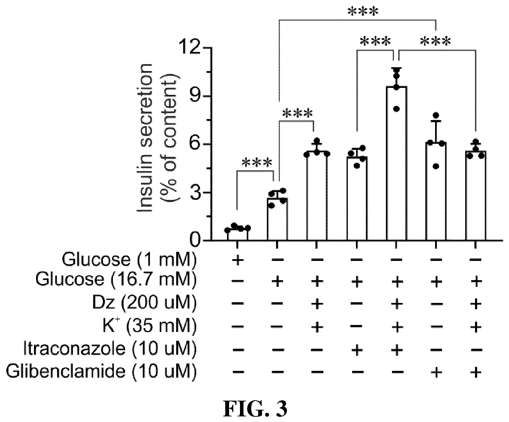

Results are shown in FIG. 3. Only Itraconazole could further increase the potentiation of glucose-stimulated insulin secretion by K⁺/diazoxide indicating different mechanism of action concerning ltraconazole- and Glibenclamide-induced potentiation of insulin secretion. Mean±SD for four in each group are shown. ***p<0.001.

## Claims

1. An agent selected from Itraconazole and Azithromycin, or a pharmaceutically acceptable salt or analogue thereof, for use in the treatment of impaired insulin secretion in a human subject.

2. The agent for use according to claim 1, wherein the human subject has a prediabetic condition that is further **characterized by** the presence of one or more of: elevated fasting blood glucose levels, elevated glucose levels in a glucose tolerance test, elevated HbA1c in blood.

3. The agent for use according to claim 1 or claim 2, wherein the human subject is diagnosed with type 2 diabetes.

4. The agent for use according to any one of the previous claims, wherein the analogue of itraconazole is selected from Voriconazole, Posaconazole, Isavuconazole, Fluconazole, Ravuconazole, Ketoconazole, Imidazoles.

5. The agent for use according to any one of the previous claims, wherein the analogue of Azithromycin is selected from clarithromycin, dirithromycin, erythromycin, carbomycin A, josamycin, kitasamycin, midecamycin, oleandomycin, spiramycin, troleandomycin, tylosin, roxithromycin and boromycin.

6. An agent selected from Itraconazole and Azithromycin, or a pharmaceutically acceptable salt or analogue thereof, for use in the treatment or prevention of type 2 diabetes in a human subject.

7. The agent for use according to the previous claim, wherein the prevention of type 2 diabetes comprises preventing the progression of a prediabetic condition to type 2 diabetes in a human subject.

8. The antifungal or antibacterial agent, or a pharmaceutically acceptable salt thereof, for use according to the previous claim, wherein the prediabetic condition in the human subject is **characterized by** the presence of one or more of: elevated fasting blood glucose levels, impaired insulin secretion, elevated glucose levels in a glucose tolerance test (above 140mg/dL 2h after administration of 75g sugar), elevated HbA1c in blood.

9. The antifungal or antibacterial agent, or a pharmaceutically acceptable salt thereof, for use according to claim 7 or claim 8, wherein preventing progression of a prediabetic condition in the human subject comprises ameliorating impaired insulin secretion in the subject.

10. The agent for use according to any one of the previous claims 6-9, wherein the analogue of itraconazole is selected from Voriconazole, Posaconazole, Isavuconazole, Fluconazole, Ravuconazole, Ketoconazole, Imidazoles.

11. The agent for use according to any one of the previous claims 6-10, wherein the analogue of Azithromycin is selected from clarithromycin, dirithromycin, erythromycin, carbomycin A, josamycin, kitasamycin, midecamycin, oleandomycin, spiramycin, troleandomycin, tylosin, roxithromycin and boromycin.
